# EUROPEAN PATENT SPECIFICATION

(11) **EP 2 717 889 B1**
(45) Date of publication and mention of the grant of the patent: **20.04.2016**
(21) Application number: 12727910.7
(22) Date of filing: 08.06.2012
(51) Int. Cl.: A61K 35/74, A61P 11/00, A61P 11/02, A61P 11/04, A61P 11/14, A61K 35/745, A61K 35/747

(54) **TREATMENT OF RESPIRATORY TRACT ILLNESS WITH BIFIDOBACTERIUM LACTIS BL-04**
BEHANDLUNG VON ATEMWEGSERKRANKUNGEN MIT BIFIDOBACTERIUM LACTIS BL-04
TRAITEMENT D'UNE MALADIE DES VOIES RESPIRATOIRES À L'AIDE DE BIFIDOBACTERIUM LACTIS BL-04

(30) Priority: 10.06.2011 US 201161495614 P; 15.06.2011 GB 201110095
(43) Date of publication of application: 16.04.2014
(73) Proprietor: DuPont Nutrition Biosciences ApS, 1001 Copenhagen K (DK)
(72) Inventor: PYNE, David, Macgregor ACT 2615 (AU); WEST, Nicholas, Queensland 4218 (AU); CRIPPS, Allan, Queensland 4226 (AU); LAHTINEN, Sampo, FIN-Siuntio 02580 (FI)
(74) Representative: DuPont EMEA
(86) International application number: PCT/GB2012/051302
(87) International publication number: WO 2012/168732

(56) References cited:
- WO-A1-2008/071930
- WO-A1-2009/071578
- WO-A2-2008/053444
- WO-A2-2008/122892
- US-A1- 2009 196 921
- OUWEHAND ARTHUR C ET AL: "Specific probiotics alleviate allergic rhinitis during the birch pollen season.", WORLD JOURNAL OF GASTROENTEROLOGY : WJG 14 JUL 2009 LNKD- PUBMED:19598302, vol. 15, no. 26, 14 July 2009 (2009-07-14) , pages 3261-3268, XP002682196, ISSN: 1007-9327

## Description

### Field of Invention

The present invention relates to *Bifidobacterium lactis BL-04* for use in the treatment of respiratory tract illnesses.

### Background

According to a 2006 report by the Australian Institute of Health and Welfare, upper respiratory tract illness URTI is the second most commonly managed problem in general practice and a large cause of hospital admissions among children (Welfare, 2006). These infections are accompanied by substantial economic burden. A quarter of all time taken off work was as a result of URTI (Leder *et al.,* 2003) while an economic analysis estimated the direct cost of respiratory tract infections at $9 billion in the US (Dixon, 1985). In many cases these illnesses reduce social interaction, health and well-being (Hashem & Hall, 2003). URTI may have increased clinical significance for sub-groups in the population. Remaining free of respiratory illness is important for athletes given evidence showing those remaining free of illness perform better than their counterparts reporting illness (Pyne *et al.,* 2001). Reducing the prevalence of these infections would appear to have substantial benefits, both economically and at the community level.

There are several placebo-controlled clinical studies that have examined the efficacy of probiotics alone (de Vrese *et al.,* 2006; Cox *et al.,* 2008) and in combination with other purported preventative agents (Pregliasco *et a*/*.,* 2008) in relation to common respiratory tract illnesses. These studies indicate that probiotic supplementation reduces the number, duration and severity of illness in comparison to placebo supplements (de Vrese *et al.,* 2005; Winkler *et al.,* 2005; de Vrese *et al.,* 2006). More recently, a study in an athletic cohort (mean age 27.0 ± 11.6 y; mean ± SD, self reported training load ∼ 10 hrs per week) reported that regular ingestion of probiotics appears to be beneficial in reducing the frequency of URTI, which may be related to better maintenance of saliva IgA levels during a winter period of training and competition. These studies indicate probiotic supplementation may be a useful nutrition product to reduce the frequency, duration and severity of common infectious illnesses.

WO2008/122892 and Ouwehand A., et al; World J. Gastroenterol. 15: 3261-3268 (2009) each disclose the use of *Bifidobacterium lactis BL-04* for the treatment of nasal/respiratory allergy symptoms, in combination with a further probiotic.

The inventors have surprisingly found that use of *Bifidobacterium lactis BL-04* (Barrangou R., et al; J. Bacteriol. 191:4144-4151(2009) provides improved probiotic effects in the treatment and prevention of respiratory tract illness when compared to other probiotic bacteria or placebo in a cohort of healthy physically active adults.

### Summary

The present invention is based on the inventors surprising finding that use of the probiotic bacteria *Bifidobacterium lactis BL-04* (available from DuPont Nutrition BioSciences Aps [formerly Danisco A/S] of Langebrogade 1, PO Box 17, DK-1001 Copenhagen K, Denmark) in the treatment and/or prophylaxis of respiratory tract illness provides beneficial results. It is particularly surprising that this bacteria has an improved therapeutic effect compared to other probiotic bacteria and mixtures of probiotic bacteria.

*Bifidobacterium lactis BL-04* is also known as *Bifidobacterium animalis* subsp. *lactis* BL-04 - these terms are used herein interchangeably. *Bifidobacterium lactis* BL-04 has also been known as DGCC2908 and RB4825.

*Bifidobacterium lactis BL-04* was originally isolated from a fecal sample from a healthy adult and is a commercial strain which has been used and deposited at the American Type Culture Collection (ATCC) safe deposit as strain SD5219 (see Barrangou R., et al; J. Bacteriol. 191:4144-4151(2009)).

This is a publically available strain.

The inventors have discovered that administration of *Bifidobacterium lactis BL-04* to healthy physically active adults results in improved outcomes in relation to severity, duration and frequency of respiratory tract illness. In the prior art, the patient groups described as benefiting from administration of probiotic bacteria have generally been those having less robust immune systems i.e. children and/or immunocompromised patients. This unexpected discovery is surprising since healthy physically active adults generally have the most robust immune system and as such only become infected by more severe or more virulent infections which are in general more difficult to combat.

Therefore, in one aspect of the present invention there is provided a composition comprising a bacterium and/or a fermentation product of a bacterium and/or a cell lysate of a bacterium for use in the treatment or prophylaxis of respiratory tract illness in a subject, wherein said bacterium is *Bifidobacterium lactis BL-04* alone.

### Detailed description

The detailed aspects of this invention are set out below. In part some of the detailed aspects are discussed in separate sections. This is for ease of reference and is in no way limiting. All of the embodiments described below are equally applicable to all aspects of the present invention unless the context specifically dictates otherwise.

As used herein the term "respiratory tract illness" refers to both illnesses of the upper and lower respiratory tracts. The term illness as used herein is synonymous with the term disorder.

In one embodiment the respiratory tract illness is an upper respiratory tract illness.

Upper respiratory tract illnesses include, for example, tonsillitis, otitis media rhinitis (inflammation of the nasal mucosa); rhinosinusitis or sinusitis (inflammation of the nares and paranasal sinuses, including frontal, ethmoid, maxillary, and sphenoid); nasopharyngitis, rhinopharyngitis or the common cold (inflammation of the nares, pharynx, hypopharynx, uvula, and tonsils); pharyngitis (inflammation of the pharynx, hypopharynx, uvula, and tonsils); epiglottitis or supraglottitis (inflammation of the superior portion of the larynx and upraglottic area); laryngitis (inflammation of the larynx); laryngotracheitis (inflammation of the larynx, trachea, and subglottic area); and tracheitis (inflammation of the trachea and subglottic area).

In one embodiment the respiratory tract illness is selected from the group consisting of one or more of the following: tonsillitis, otitis media rhinitis (inflammation of the nasal mucosa); rhinosinusitis or sinusitis (inflammation of the nares and paranasal sinuses, including frontal, ethmoid, maxillary, and sphenoid); nasopharyngitis, rhinopharyngitis or the common cold (inflammation of the nares, pharynx, hypopharynx, uvula, and tonsils); pharyngitis (inflammation of the pharynx, hypopharynx, uvula, and tonsils); epiglottitis or supraglottitis (inflammation of the superior portion of the larynx and upraglottic area); laryngitis (inflammation of the larynx); laryngotracheitis (inflammation of the larynx, trachea, and subglottic area); and tracheitis (inflammation of the trachea and subglottic area).

In an embodiment of the invention the respiratory tract illness is selected from the group consisting of one or more of the following: throat soreness, sneezing, blocked nose, runny nose and cough.

One or more of throat soreness, sneezing, blocked nose, runny nose or a cough is a symptom of an upper respiratory tract illness.

In one embodiment the composition according to the present invention can be used for the treatment or prophylaxis of two or more (including three or more; or four or more; or all) of throat soreness, sneezing, blocked nose, runny nose or a cough is a symptom of an upper respiratory tract illness.

One or more of throat soreness, sneezing, blocked nose, runny nose or a cough is a symptom of one or more of the group consisting of: tonsillitis, otitis media rhinitis (inflammation of the nasal mucosa); rhinosinusitis or sinusitis (inflammation of the nares and paranasal sinuses, including frontal, ethmoid, maxillary, and sphenoid); nasopharyngitis, rhinopharyngitis or the common cold (inflammation of the nares, pharynx, hypopharynx, uvula, and tonsils); pharyngitis (inflammation of the pharynx, hypopharynx, uvula, and tonsils); epiglottitis or supraglottitis (inflammation of the superior portion of the larynx and upraglottic area); laryngitis (inflammation of the larynx); laryngotracheitis (inflammation of the larynx, trachea, and subglottic area); and tracheitis (inflammation of the trachea and subglottic area).

In one embodiment the respiratory tract illness is a lower respiratory tract illness.

Lower respiratory tract illnesses include, for example, bronchitis, acute bronchitis, pneumonia, lung abscesses.

In an embodiment of the invention the respiratory tract illness is selected from the group consisting of one or more of the following: coughing with chest congestion and coughing with wheezing.

In one embodiment of the invention the respiratory tract illness is selected from the group consisting of both of the following: coughing with chest congestion and coughing with wheezing.

One or more of coughing with chest congestion and coughing with wheezing is a symptom of a lower respiratory tract illness.

One or more of coughing with chest congestion and coughing with wheezing is a symptom of one or more the group consisting of bronchitis, acute bronchitis, pneumonia, and lung abscesses.

In one embodiment the present invention relates to reducing one or more of the incidence, duration or severity (preferably the incidence) of respiratory tract illness, e.g. upper respiratory tract illness or lower respiratory tract illness.

In another embodiment the present invention relates to reducing the incidence, duration or severity (preferably the incidence) of the symptoms of respiratory tract illness, e.g. upper respiratory tract illness or lower respiratory tract illness.

Preferably the reduction in the incidence (number or frequency), duration or severity of respiratory tract illness or of the symptoms of respiratory tract illness is following administration of *Bifidobacterium lactis* BL-04 compared with the incidence (number or frequency), duration or severity, respectively, of respiratory tract illness or of the symptoms of respiratory tract illness in comparative subjects without administration of *Bifidobacterium lactis* BL-04.

In one embodiment the composition is particularly effective in reducing the incidence, duration or severity (preferably the incidence) of the respiratory tract illness in subjects which display symptoms for more than 7 days. Without wishing to be bound by theory, if symptoms are displayed for more than 7 days this is indicative of a severe respiratory tract illness. In one embodiment, the present composition is particularly effective in reducing the incidence, duration or severity (preferably the incidence) of severe respiratory tract illness.

In one embodiment, the term "incidence" refers to the number and/or frequency of respiratory tract illnesses or symptoms thereof. In a further embodiment, the term "incidence" refers to the number and/or frequency of respiratory tract illnesses or symptoms thereof lasting at least three days or at least five days or at least 7 days.

In one embodiment, "prophylaxis" is in relation to respiratory tract illness or symptoms thereof lasting at least three days or at least five days or at least 7 days. In a further embodiment, "prophylaxis" is in relation to severe respiratory tract illness or symptoms thereof.

The term "a fermentation product of *Bifidobacterium lactis BL-04"* as used herein means a composition which results from culturing (e.g. fermenting) *Bifidobacterium lactis BL-04* in a suitable media; or a supernatant or a fraction or a component thereof. In one embodiment the fermentation product of *Bifidobacterium lactis BL-04* is the whole composition which results from culturing (e.g. fermenting) *Bifidobacterium lactis BL-04* in a suitable media. The fermentation product may be dried prior to use.

The fermentation product of *Bifidobacterium lactis BL-04* in one embodiment may comprise viable *Bifidobacterium lactis BL-04.* The fermentation product of *Bifidobacterium lactis BL-04* in another embodiment may be a cell-free fermentation product. A cell-free fermentation product may be a fermentation product of *Bifidobacterium lactis BL-04* which results from culturing (e.g. fermenting) *Bifidobacterium lactis BL-04* in a suitable media, which has been modified to remove and/or to inactive the bacterial cells to provide a cell-free fermentate. In another embodiment the fermentation product of *Bifidobacterium lactis BL-04* may comprise non-viable *Bifidobacterium lactis BL-04* which may be whole or lysated.

The term "cell-free" as used herein means that the fermentation product (preferably the fermentate) is substantially free of viable bacterial cells, typically containing less than about 10⁵ viable bacterial cells/mL fermentation product, less than about 10⁴ viable bacterial cells/mL fermentation product, less than about 10³ viable bacterial cells/mL fermentation product, less than about 10² viable bacterial cells/mL fermentation product, or less than about 10 viable bacterial cells/mL fermentation product. Preferably, the fermentation product is substantially free of cells, typically containing less than about 10⁵ cells/mL fermentation product, less than about 10⁴ cells/mL fermentation product, less than about 10³ cells/mL fermentation product, less than about 10² cells/mL fermentation product, or less than about 10 cells/mL fermentation product.

In one aspect, one or more cells may be separated from the fermentation product (e.g., fermentate). Such separation may be achieved by any means known in the art including by centrifuging and/or filtering. For example, the fermentation product can be filtered (one or several times in a multistep process) to remove such components as particulate matter, cells and the like. Alternatively or in addition, one or more cells and/or one of more spores may be separated from the fermentation product (e.g. fermentate) by centrifugation, thus producing a supernatant. Depending on the speed and duration of the centrifugation, the supernatant can be cell free (i.e., a cell-free supernatant) or the supernatant can contain cells, which can be filtered or further centrifuged to provide a cell-free supernatant.

In some aspects the fermentation product of *Bifidobacterium lactis BL-04* may be a crude extract of the culture medium.

In some aspects the fermentation product of *Bifidobacterium lactis BL-04* may comprise a mixture of constituents present following (e.g. at the end of) the culturing of *Bifidobacterium lactis BL-04.* Hence, the term fermentation product may comprise in addition to active ingredients other components such as particulate matter, solids, substrates not utilised during culturing, debris, media, and cell waste.

The term "cell-lysate of *Bifidobacterium lactis BL-04"* as used herein means the cellular debris and fluid produced by lysis of a *Bifidobacterium lactis BL-04* cell(s). Preferably the *Bifidobacterium lactis BL-04* cell(s) is/are isolated before being lysed.

Preferably the *Bifidobacterium lactis* BL-04 cells are lysed by the following method: *Bifidobacterium lactis* BL-04 are cultured at 37°C anaerobically in MRS (or another suitably culture medium) supplemented with 0.05% cysteine; the bacterial cells are harvested by centrifugation (6000 rpm/5min); the supernatant is aspirated and the pellet is optionally frozen at -70°C; 1.5 ml pellet of bacterial cell culture to which 150 µl of T10E1 is added (10 mM Tris-HCl, pH 7.5; 1 mM EDTA) and vortexed to resuspend the cell pellet; 1 µl of Ready-Lyse™ Lysozyme (Epicentre, Vol 10, No. 3, 2003) is added to each resuspended pellet of bacteria (from 1 to 1.5 ml of culture); incubation at 37°C for 30 minutes to overnight; 1 µl of Proteinase K (50 µg/µl) is diluted into 150 µl of 2X T&C Lysis Solution (both are provided in the MasterPure DNA Purification Kit, or sold separately) for each 1 to 1.5 ml of culture pelleted; 150 µl of the Proteinase K/Lysis solution is added to the sample and mixed thoroughly; incubation at 65°C to 70°C for 15 minutes, briefly vortexing every 5 minutes; cooling the samples to 37°C; 1 µl of RNase A (5 µg/µl, provided in the kit, or sold separately) is added to each sample and mixed thoroughly; incubation at 37°C for 30 minutes; the samples are placed on ice.

Alternative lysis methods for gram positive bacteria may be used to lyse the *Bifidobacterium lactis BL-04 -* there are known to one skilled in the art.

Suitably, the fermentation product of *Bifidobacterium lactis BL-04* may be a fermentation product which is present in the supernatant phase isolated from a culture of the *Bifidobacterium lactis BL-04* cultured under the following conditions: 37°C anaerobically in MRS (or another suitably medium) supplemented with 0.05% cysteine.

In one embodiment, the fermentation product may be obtainable (preferably obtained) by culturing the bacterium in a culture medium until the OD of the culture at λ600 reaches at least 0.6, preferably 0.6 to 1.5; optionally removing the bacteria by centrifugation and/or filtration (such as, for example, centrifugation at 25°C, 5 min, 3000g and/or sterile-filtration) to result in a cell-free fermentate product comprising active ingredient(s).

Suitably, the fermentation product is obtainable (preferably obtained) using an MRS culture medium either with 1.0% sugar or without sugar. Suitably, the fermentation product is obtainable (preferably obtained) by culturing the bacteria at 37°C. Suitably, the fermentation product is obtainable (preferably obtained) by culturing the bacteria anaerobically.

The culturing of *Bifidobacterium lactis BL-04* can take place from about 1 to about 72 hours (h), from about 5 to about 60 h, or from about 10 to about 54 h or from 24 to 48 h.

In one aspect, the culturing can be carried out until nutrient depletion (preferably complete nutrient) occurs.

In one aspect, the culturing is for a time effective to reach the stationary phase of growth of the bacteria.

The temperature during the culturing can be from about 30 to about 50 °C from about 32 to about 40 °C, or from about 34 to about 38 °C, or at about 37°C.

The pH during the culturing can be at a pH from about 5 to about 9, from about 5 to about 6, from about 6 to about 7, from about 7 to about 8,.

In one aspect, the culturing preferably takes place under aeration.

Batch and continuous culturing are known to a person of ordinary skill in the art. The fermentation product of the present invention may be prepared using batch or continuous culturing.

Suitably, the fermentation product may be harvested during or at the end of the culturing process

In one aspect, the fermentation product of the present invention is harvested during or at the end of the exponential phase.

In one aspect, the fermentation product of the present invention is harvested at or during the stationary phase.

In one aspect of the present invention, the fermentation product may be produced in a vat under commercial conditions.

In one aspect, the culture is agitated and/or stirred during culturing (e.g. during fermentation).

In one aspect, the level of oxygenation is monitored and/or controlled during the culturing.

Suitably, the composition comprising *Bifidobacterium lactis BL-04* according to the present invention or the fermentation product of *Bifidobacterium lactis BL-*04 or cell lysate of Bifidobacterium lactis BL-04 may be in the form of a bacterial suspension, before or after freezing, or in the form of concentrates, either in dry, lyophilized or frozen form. Whatever the form used, the strain can be frozen.

The composition comprising *Bifidobacterium lactis BL-04* according to the present invention does not comprise a further microorganism, e.g. does not comprise a further probiotic bacterium.

In one embodiment the composition according the present invention consists of *Bifidabacterium lactis BL-04,* e.g. together with excipients, diluents or carriers.

Suitably, the composition comprising *Bifidobacterium lactis BL-04* and/or a fermentation product of *Bifidobacterium lacfis BL-04* and/or a cell lysate of *Bifidobacterium lactis BL-04* according to the present invention may contain one or more additives. Suitably additives may be added during drying and/or during lyophilisation of the composition.

The composition comprising *Bifidobacterium lactis BL-04* used in accordance with the present invention may comprise from 10⁶ to 10¹² CFU of bacteria/g of composition, and more particularly from 10⁸ to 10¹² CFU of bacteria/g of composition, preferably 10⁹ to 10¹² CFU/g for the lyophilized form.

Suitably the composition comprising *Bifidobacterium lactis BL-04* used in accordance with the present invention may be administered at a dosage of from about 10⁶ to about 10¹² CFU of *Bifidobacterium lacfis BL-04*/dose, preferably about 10⁸ to about 10¹² CFU of *Bifidobacterium lactis BL-04*/dose. By the term "per dose" it is meant that this amount of *Bifidobacterium lactis BL-04* is provided to a subject either per day or per intake, preferably per day. For example, if the *Bifidobacterium lactis BL-04* is to be administered in a food (for example in a yoghurt) - then the yoghurt will preferably contain from about 10⁸ to 10¹² CFU of the *Bifidobacterium lactis BL-04.* Alternatively, however, this amount of *Bifidobacterium lactis BL-04* may be split into multiple administrations each consisting of a smaller amount of microbial loading - so long as the overall amount of *Bifidobacterium lactis BL-04* received by the subject in any specific time (for instance each 24h period) is from about 10⁶ to about 10¹² CFU of *Bifidobacterium lactis BL-04,* preferably 10⁸ to about 10¹² CFU of *Bifidobacterium lactis BL-04.*

In accordance with the present invention an effective amount of *Bifidobacterium lactis BL-04* may be at least 10⁶ CFU of microorganism/dose, preferably from about 10⁶ to about 10¹² CFU of microorganism/dose, preferably about 10⁸ to about 10¹² CFU of microorganism/dose.

In one embodiment, preferably the *Bifidobacterium lactis BL-04* used in accordance with the present invention may be administered at a dosage of from about 10⁶ to about 10¹² CFU of microorganism/day, preferably about 10⁸ to about 10¹² CFU of microorganism/day. Hence, the effective amount in this embodiment may be from about 10⁶ to about 10¹² CFU of microorganism/day, preferably about 10⁸ to about 10¹² CFU of microorganism/day.

In one embodiment, preferably the *Bifidobacterium lactis* BL-04 used in accordance with the present invention is administered at a dosage of from about 10⁸ to about 10¹⁰ CFU/day, preferably about 1x10⁹ to 3x10⁹ CFU/day, for example about 2x10⁹ CFU of microorganism/day

CFU stands for "colony-forming units".

The composition comprising *Bifidobacterium lactis BL-04* and/or a fermentation product of *Bifidobacterium lactis BL-04* and/or a cell lysate of *Bifidobacterium lactis BL-04* according to the present invention may be used to modify medication (e.g. cold and/or flu medication) intake in a subject.

In one embodiment the term "modify" means "reduce".

The modification and/or reduction of medication means a modification or reduction compared with a placebo control.

The placebo control is not administered a composition comprising *Bifidobacterium lactis BL-04* and/or a fermentation product of *Bifidobacterium lactis BL-04* and/or a cell lysate of *Bifidobacterium lactis BL-04* according to the present invention. The placebo control may be administered sucrose.

The term "medication" as used herein preferably means cold and/or flu medication. This may include one or more of the following types of medication: sore throat treatment, catarrh treatment, cough treatments, decongestants, anti-histamines, ferver reducers, pain relievers, cough suppressants (antitussives), antibiotics, or expectorants (for thinning mucus).

The medication may comprise or be one or more of the following: a decongestant, an antihistamine, fever reducer, pain reliever (e.g. for headaches and/or other aches and pains), non-steroidal anti-inflammatory drugs (NSAIDs), antitussives (or cough suppressants), an antibiotic, or expectorants (for thinning mucus).

The pain reliever may include one or more of the following active ingredients: paracetamol, aspirin, ibuprofen for example.

The fever reducer may include one or more of the following active ingredients: aspirin, ibuprofen, magnesium salicylate, naproxen for example.

The decongestant may include one or more of the following active ingredients: phenylpropanolamine (PPA) and/or pseudo-ephedrine and/or phenylephrine for example.

The cough suppressant (or antitrussive) may comprise pholcodine and/or noscoapine) for example.

The expectorant may include one or more of the following active ingredients: guaifenesin, acetylcysteine or ambroxol for example.

The anti-histamine may include one or more of the following active ingredients: brompheniramine; chlorpheniramine, dimenhydrinate, diphenhydramine, doxylamine; loratadine; cetirizine or fexofenadine for example.

The antibiotic may include one or more of the following active ingredients: azithromycin, clarithromycin, dirithromycin, erythromycin, roxithromycin, troleandomycin, penicillin, amoxicillin, rimacillin, or ampicillin.

The term "medication intake" as used herein means the total number of (cold and/or flu) medications taken by a subject and/or the total number of days a subject uses (cold and/or flu) medication.

The composition comprising *Bifidobacterium lactis BL-04* and/or a fermentation product of *Bifidobacterium lactis BL-04* and/or a cell lysate of *Bifidobacterium lactis BL-04* according to the present invention may be used to reduce the total number of cold and/or flu medication taken by a subject and/or reduce the total number of days a subject uses cold and/or flue medication (e.g. compared with a placebo control).

The composition comprising *Bifidobacterium lactis BL-04* and/or a fermentation product of *Bifidobacterium lactis BL-04* and/or a cell lysate of *Bifidobacterium lactis BL-04* according to the present invention maybe administered in or as a food product or may be administered as a pharmaceutically acceptable composition.

In one embodiment, the present invention results in an increase in the granulocyte (neutrophil) phagocytic activity in the subject. Preferably, this is increase in activity is at least 1%, at least 3%, at least 5%, at least 7%, at least 10%, at least 12%, at least 15%, at least 17%, at least 20%, at least 25%, at least 30%, at least 40% or at least 50%.

In one embodiment, the present invention results in an increase in the monocyte phagocytic activity in the subject. Preferably, this is increase in activity is at least 1%, at least 3%, at least 5%, at least 7%, at least 10%, at least 12%, at least 15%, at least 17%, at least 20%, at least 25%, at least 30%, at least 40% or at least 50%.

In this specification the term 'probiotic microorganism' is defined as covering any non-pathogenic microorganism which, when administered live (e.g. viable) in adequate amounts, confer a health benefit on the host. These probiotic strains generally have the ability to survive the passage through the upper part of the digestive tract. They are non-pathogenic, non-toxic and exercise their beneficial effect on health on the one hand via ecological interactions with the resident flora in the digestive tract, and on the other hand via their ability to influence the immune system in a positive manner via the "GALT" (gut-associated lymphoid tissue). Depending on the definition of probiotics, these microorganism, when given in a sufficient number, have the ability to progress live through the intestine, however they do not cross the intestinal barrier and their primary effects are therefore induced in the lumen and/or the wall of the gastrointestinal tract. They then form part of the resident flora during the administration period. This colonization (or transient colonization) allows the probiotic microorganism to exercise a beneficial effect, such as the repression of potentially pathogenic micro-organisms present in the flora and interactions with the immune system of the intestine.

It will be understood that the composition according to the present invention may be formulated as a medicament, a food product or a dietary supplement. Preferably, the composition is formulated as a dietary supplement.
Advantageously, where the product is a foodstuff, the *Bifidobacterium lactis BL-04* and/or a fermentation product of *Bifidobacterium lactis BL-04* and/or a cell lysate of *Bifidobacterium lactis BL-04* remain effective (e.g. the bacteria remain viable) through the normal "sell-by" or "expiration" date during which the food product is offered for sale by the retailer. Preferably, the effective time should extend past such dates until the end of the normal freshness period when food spoilage becomes apparent. The desired lengths of time and normal shelf life will vary from foodstuff to foodstuff and those of ordinary skill in the art will recognise that shelf-life times will vary upon the type of foodstuff, the size of the foodstuff, storage temperatures, processing conditions, packaging material and packaging equipment.
Preferably, the present invention results in the modulation of expression of at least one cytokine. Preferably, the cytokine is selected from the group consisting of GM-CSF, IL-1RA, IL-6, IL-8, IL10, TNF-α and INF- y.

In one embodiment preferably the *Bifidobacterium lactis BL-04* in the composition and/or the fermentation product of the present invention are viable.
The term "viable" means a microorganism (bacterium) is metabolically active or able to differentiate.
In a preferred embodiment the composition comprises viable *Bifidobacterium lactis* BL04.
In some embodiments the *Bifidobacterium lactis* BL04 (e.g. viable cells) are isolated from the medium in which it was cultured or the fermentation product prior to forming the composition comprising *Bifidobacterium lactis* BL04 of the present invention.

### Subject

The term "subject", as used herein, means an animal. Preferably, the subject is a mammal, including for example livestock (including cattle, horses, pigs, chickens and sheep), and humans. In some aspects of the present invention the animal is a companion animal (including pets), such as a dog or a cat for instance. In some aspects of the present invention, the subject may suitably be a human.
In one embodiment the subject is a human.
In one embodiment the subject may be female.
In one embodiment the subject may be male.
In one embodiment the subject is not a child. The term "child" as used herein means a human 7 years of age or younger.
In one embodiment the subject is a human that is 8 years of age or older.
In one embodiment the subject is a human that is 16 years of age or older.
In one embodiment the subject is a human that is 18 years of age or older.
In one embodiment the subject is not an immunocompromised subject.
In one embodiment the subject is a healthy subject.
In one embodiment the subject is not an exercise-induced immunosuppressed subject.
In one embodiment the subject is not an athlete, e.g. is not an elite athlete, e.g. is not a distance runner.

In one embodiment the subject is a healthy, physically active adult. Adult as used herein may mean a human that is 18 years of age or older.
In one embodiment the subject has a fully developed and non-compromised immune system.

### Advantages

The inventors have surprisingly found that use of *Bifidobacterium lactis BL-04* significantly reduces the incidence, duration and/or severity of respiratory tract illness (e.g. URTI and/or lower respiratory tract illness) or the symptoms of respiratory tract illness when compared to other probiotic bacteria or a placebo in healthy physically active adults.

Surprisingly this effect has been found using a single strain of *Bifidobacterium* (e.g. alone or without a further microorganism in the composition).

One advantage of the present invention is that *Bifidobacterium lactis BL-04* significantly reduces the incidence, duration and/or severity of both URTI and lower respiratory tract illness; or significantly reduces the incidence, duration and/or severity of the symptoms of both URTI and lower respiratory tract illness.

This is the first time that a strain of *Bifidobacterium* has been shown to have an effect on both URTI and lower respiratory tract illness or on the symptoms of both URTI and lower respiratory tract illness.

Importantly the subjects are healthy physically active adults. This contrasts with previous studies which have been performed in children or immunosuppressed individuals (i.e. in individuals where their immune system is either not fully developed or in some way challenged). Some studies have focussed on elite athletes (e.g. distance runners) - again where the individuals undergo strenuous training and suffer from exercise-induced immunosuppression.

In the present case the inventors have surprisingly found beneficial effects in non-immunosuppressed individuals.

In addition the present inventors have surprisingly found that *Bifidobacterium lactis BL-04* can significantly reduces the incidence, duration and/or severity of respiratory tract illness (e.g. URTI and/or lower respiratory tract illness) or the symptoms of respiratory tract illness even when not in a synbiotic composition or a composition with vitamins and/or minerals.

The present inventors have found that the advantageous effects observed herein can be obtained using *Bifidobacterium lactis BL-04* alone. This can lead to many advantages including simplifying the production of the supplement and/or reducing costs of the manufacture of the supplement and the supplement itself.

A further advantage is that *Bifidobacterium lactis BL-04* is used without additional probiotic bacteria - this has the advantage that it simplifies the stability issues with regard to the supplement. Therefore one advantage of the present invention is that the composition comprising the *Bifidobacterium lactis BL-04* (e.g. alone or without the presence of another microorganism) is easier to stabilise as the skilled person is only concerned with the stability of a single bacterium rather than more than one bacterium.

In addition or alternatively the use of a single bacterial strain in a composition can have the advantage of reducing cost-in-use of the composition compared with mixed strain compositions.

A significant further advantage described herein is that the inventors have surprising found that use of a composition (comprising *Bifidobacterium lactis BL-04)* in accordance with the present invention can be used to modify or reduce the medication intake in a subject.

Thus the composition described herein advantageously can lead to a reduction in medication use by an individual. This includes reducing the amount of one or more of the following medicaments used by the individual: a decongestant, an antihistamine, fever reducer, pain reliever (e.g. for headaches and/or other aches and pains), non-steroidal anti-inflammatory drugs (NSAIDs), antitussives (or cough suppressants), an antibiotic, or expectorants (for thinning mucus).

### Medicament

The term "medicament" as used herein in relation to the invention encompasses medicaments for both human and animal usage in human and veterinary medicine. In addition, the term "medicament" as used herein means any substance which provides a therapeutic and/or beneficial effect. The term "medicament" as used herein is not necessarily limited to substances which need Marketing Approval, but may include substances which can be used in cosmetics, nutraceuticals, food (including feeds and beverages for example) and natural remedies.

### Treatment

It is to be appreciated that all references herein to treatment include curative, palliative and prophylactic treatment.

### Substantially pure form and/or isolated form

For some aspects the microorganism and/or fermentation product and/or cell lysate according to the present invention may be in a substantially pure form or may be in an isolated form.
The term "substantially pure form" is used to indicate that the microorganism and/or fermentation product and/or cell lysate according to the present invention is present at a high level. When the microorganism and/or fermentation product and/or cell lysate is in a substantially pure form, the microorganism and/or fermentation product and/or cell lysate is desirably the predominant component present in a composition. Preferably it is present at a level of more than 30%, of more than 50%, of more than 75%, of more than 90%, or even of more than 95%, said level being determined on a dry weight / dry weight basis with respect to the total composition under consideration.

At very high levels (e.g. at levels of more than 90 %, of more than 95% or of more than 99%) the microorganism and/or fermentation product and/or cell lysate may be regarded as being "isolated".

Biologically active substances of the present invention (including polypeptides, nucleic acid molecules, carbohydrates identified/identifiable via screening, lipids identified/identifiable via screening, moieties identified/identifiable via screening, etc.) may be provided in a form that is substantially free of one or more contaminants with which the substance might otherwise be associated. Thus, for example, they may be substantially free of one or more potentially contaminating polypeptides and/or nucleic acid molecules. They may be provided in a form that is substantially free of other cell components (e.g. of cell membranes, of cytoplasm, etc.). When a composition is substantially free of a given contaminant, the contaminant will be at a low level (e.g. at a level of less than 10%, less than 5% or less than 1% on the dry weight/dry weight basis set out above).

### Combination with other components

The *Bifidobacterium lactis BL04* and/or fermentation product thereof and/or cell lysate thereof for use in the present invention may be used in combination with other components. Thus, the present invention also relates to combinations. The *Bifidobacterium lactis BL04* and/or fermentation product thereof and/or cell lysate thereof may be referred to herein as "the composition of the present invention".
The combination of the present invention comprises the composition of the present invention and another component which is suitable for animal or human consumption and is capable of providing a medical or physiological benefit to the consumer.
Other components of the combinations of the present invention include polydextrose, such as Litesse®, and/or a maltodextrin and/or lactitol. These other components may be optionally added to the composition to assist the drying process and help the survival of the microorganisms.
Further examples of other suitable components include one or more of: thickeners, gelling agents, emulsifiers, binders, crystal modifiers, sweeteners (including artificial sweeteners), rheology modifiers, stabilisers, anti-oxidants, dyes, enzymes, carriers, vehicles, excipients, diluents, lubricating agents, flavouring agents, colouring matter, suspending agents, disintegrants, granulation binders etc. These other components may be natural. These other components may be prepared by use of chemical and/or enzymatic techniques.
In one embodiment the microorganism and/or fermentation product and/or cell lysate thereof may be encapsulated.
In one preferred embodiment the microorganism and/or fermentation product and/or cell lysate thereof for use in the present invention may be used in combination with one or more lipids.
For example, the microorganism and/or fermentation product and/or cell lysate thereof for use in the present invention may be used in combination with one or more lipid micelles. The lipid micelle may be a simple lipid micelle or a complex lipid micelle.
The lipid micelle may be an aggregate of orientated molecules of amphipathic substances.
The lipid micelles may be an aggregate, of colloidal dimensions, of orientated molecules of amphipathic substances existing in equilibrium in solution with the chemical species from which it is formed. Micelles are generally electrically charged. In aqueous solution the individual molecules of the micellar aggregate are oriented with their polar groups pointing towards the aqueous medium and their hydrophobic moiety directed into the centre of the micelle.
The lipid micelles may comprise a lipid and/or an oil.
As used herein the term "thickener or gelling agent" refers to a product that prevents separation by slowing or preventing the movement of particles, either droplets of immiscible liquids, air or insoluble solids. Thickening occurs when individual hydrated molecules cause an increase in viscosity, slowing the separation. Gelation occurs when the hydrated molecules link to form a three-dimensional network that traps the particles, thereby immobilising them.
The term "stabiliser" as used here is defined as an ingredient or combination of ingredients that keeps a product (e.g. a food product) from changing over time.

The term "emulsifier" as used herein refers to an ingredient (e.g. a food product ingredient) that prevents the separation of emulsions. Emulsions are two immiscible substances, one present in droplet form, contained within the other. Emulsions can consist of oil-in-water, where the droplet or dispersed phase is oil and the continuous phase is water; or water-in-oil, where the water becomes the dispersed phase and the continuous phase is oil. Foams, which are gas-in-liquid, and suspensions, which are solid-in-liquid, can also be stabilised through the use of emulsifiers. Aeration can occur in a three-phase system where air is entrapped by liquid oil then stabilised by agglomerated fat crystals stabilised with an emulsifier. Emulsifiers have a polar group with an affinity for water (hydrophilic) and a non-polar group which is attracted to oil (lipophilic). They are absorbed at the interfaces of the two substances, providing an interfacial film acting to stabilise the emulsion. The hydrophilic/lipophilic properties of emulsifiers are affected by the structure of the molecule. These properties are identified by the hydrophilic/lipophilic balance (HLB) value. Low HLB values indicate greater lipophilic tendencies which are used to stabilise water-in-oil emulsions. High HLB values are assigned to hydrophilic emulsifiers, typically used in oil-in-water emulsions. These values are derived from simple systems. Because foods often contain other ingredients that affect the emulsification properties, the HLB values may not always be a reliable guide for emulsifier selection.
As used herein the term "binder" refers to an ingredient (e.g. a food ingredient) that binds the product together through a physical or chemical reaction. During "gelation" for instance, water is absorbed, providing a binding effect. However, binders can absorb other liquids, such as oils, holding them within the product. In the context of the present invention binders would typically be used in solid or low-moisture products for instance baking products: pastries, doughnuts, bread and others.
The term "crystal modifier" as used herein refers to an ingredient (e.g. a food ingredient) that affects the crystallisation of either fat or water. Stabilisation of ice crystals is important for two reasons. The first is directly related to the product stability from a separation standpoint. The more freeze/thaw cycles a product encounters, the larger the ice crystals become. These large crystals can break down product structure, either naturally occurring, as in the case of cell walls, or that which is created by "elation". Because the water is no longer held in place, the product may exhibit syneresis, or weeping, after thawing. Secondly, in the case of a product which is consumed frozen, these large crystals result in an undesirable, gritty mouth feel.
"Carriers" or "vehicles" mean materials suitable for compound administration and include any such material known in the art such as, for example, any liquid, gel, solvent, liquid diluent, solubilizer, or the like, which is non-toxic and which does not interact with any components of the composition in a deleterious manner.
Examples of nutritionally acceptable carriers include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.
Examples of excipients include one or more of: microcrystalline cellulose and other celluloses, lactose, sodium citrate, calcium carbonate, dibasic calcium phosphate, glycine, starch, milk sugar and high molecular weight polyethylene glycols.
Examples of disintegrants include one or more of: starch (preferably corn, potato or tapioca starch), sodium starch glycollate, croscarmellose sodium and certain complex silicates.
Examples of granulation binders include one or more of: polyvinylpyrrolidone, hydroxypropylmethylcellulose (HPMC), hydroxypropylcellulose (HPC), sucrose, maltose, gelatin and acacia.
Examples of lubricating agents include one or more of: magnesium stearate, stearic acid, glyceryl behenate and talc.
Examples of diluents include one or more of: water, ethanol, propylene glycol and glycerin, and combinations thereof.
The other components may be used simultaneously (e.g. when they are in admixture together or even when they are delivered by different routes) or sequentially (e.g. they may be delivered by different routes).
Preferably, when the composition of the present invention when admixed with any other components, the microorganisms remain viable.

As used herein the term "component suitable for animal or human consumption" means a compound which is or can be added to the composition of the present invention as a supplement which may be of nutritional benefit, a fibre substitute or have a generally beneficial effect to the consumer. The ingredients can be used in a wide variety of products that require gelling, texturising, stabilising, suspending, film-forming and structuring, retention of juiciness, without adding unnecessary viscosity. Preferably, the ingredients will be able to improve the shelf life and stability of the viable culture.
The components may be prebiotics such as alginate, xanthan, pectin, locust bean gum (LBG), inulin, guar gum, galacto-oligosaccharide (GOS), fructo-oligosaccharide (FOS), polydextrose (i.e. Litesse®), lactitol, lactosucrose, soybean oligosaccharides, palatinose, isomalto-oligosaccharides, gluco-oligosaccharides and xylo-oligosaccharides.
The optimum amount of the composition to be used in the combination of the present invention will depend on the product to be treated and/or the method of contacting the product with the composition and/or the intended use for the same. The amount of viable microorganism used in the compositions should be a sufficient amount to be effective and to remain sufficiently effective in improving the aroma, flavour, mildness, consistency, texture, body, mouth feel, viscosity, structure and/or organoleptic properties, nutrition and/or health benefits of food products containing said composition. This length of time for effectiveness should extend up to at least the time of utilisation of the product.

### Concentrates

The compositions for use in the present invention may be in the form of concentrates. Typically these concentrates comprise a substantially high concentration of a *Bifidobacterium lactis BL04,* (e.g. viable *Bifidobacterium lactis BL04)* and/or fermentation product and/or cell lysate thereof.
Powders, granules and liquid compositions in the form of concentrates may be diluted with water or resuspended in water or other suitable diluents, for example, an appropriate growth medium such as milk or mineral or vegetable oils, to give compositions ready for use.

The combinations of the present invention in the form of concentrates may be prepared according to methods known in the art.
In one aspect of the present invention the product is contacted by a composition in a concentrated form. Preferably, the product is contacted by a spray-dried and/or resuspended composition.
The compositions of the present invention may be spray-dried or freeze-dried by methods known in the art.
Typical processes for making particles using a spray drying process involve a solid material which is dissolved in an appropriate solvent (e.g. a culture of a micro-organism in a fermentation medium). Alternatively, the material can be suspended or emulsified in a non-solvent to form a suspension or emulsion. Other ingredients (as discussed above) or components such as anti-microbial agents, stabilising agents, dyes and agents assisting with the drying process may optionally be added at this stage.
The solution then is atomised to form a fine mist of droplets. The droplets immediately enter a drying chamber where they contact a drying gas. The solvent is evaporated from the droplets into the drying gas to solidify the droplets, thereby forming particles. The particles are then separated from the drying gas and collected.

### Products

Any product which can benefit from the composition may be used in the present invention. These include but are not limited to dairy foods and dairy food-derived products, dietary supplements and pharmaceutical products.
By way of example, the composition of the present invention can be used as an ingredient to soft drinks, a fruit juice or a beverage comprising whey protein, health teas, cocoa drinks, milk drinks and lactic acid bacteria drinks, yoghurt and drinking yoghurt, cheese, ice cream, water ices and desserts, confectionery, biscuits cakes and cake mixes, snack foods, balanced foods and drinks, fruit fillings, care glaze, chocolate bakery filling, cheese cake flavoured filling, fruit flavoured cake filling, cake and doughnut icing, instant bakery filling creams, fillings for cookies, ready-to-use bakery filling, reduced calorie filling, adult nutritional beverage, acidified soy/juice beverage, aseptic/retorted chocolate drink, bar mixes, beverage powders, calcium fortified soy/plain and chocolate milk, calcium fortified coffee beverage.
The composition can further be used as an ingredient in food products such as American cheese sauce, anti-caking agent for grated & shredded cheese, chip dip, cream cheese, dry blended whip topping fat free sour cream, freeze/thaw dairy whipping cream, freeze/thaw stable whipped tipping, low fat and light natural cheddar cheese, low fat Swiss style yoghurt, aerated frozen desserts, hard pack ice cream, label friendly, improved economics & indulgence of hard pack ice cream, low fat ice cream: soft serve, barbecue sauce, cheese dip sauce, cottage cheese dressing, dry mix Alfredo sauce, mix cheese sauce, dry mix tomato sauce and others.
For certain aspects, preferably the present invention may be used in connection with yoghurt production, such as fermented yoghurt drink, yoghurt, drinking yoghurt, cheese, fermented cream, milk based desserts and others. Suitably, the composition can be further used as an ingredient in one or more of cheese applications, meat applications, or applications comprising protective cultures.
Also described herein is a method of preparing a food or a food ingredient, the method comprising admixing the composition according to the present invention with another food ingredient.
Advantageously, described herein are products that have been contacted with the composition of the present invention (and optionally with other components/ingredients), wherein the composition is used in an amount to be capable of improving the nutrition and/or health benefits of the product.
As used herein the term "contacted" refers to the indirect or direct application of the composition of the present invention to the product. Examples of the application methods which may be used, include, but are not limited to, treating the product in a material comprising the composition, direct application by mixing the composition with the product, spraying the composition onto the product surface or dipping the product into a preparation of the composition.
Where the product described herein is a foodstuff, the composition of the present invention is preferably admixed with the product. Alternatively, the composition may be included in the emulsion or raw ingredients of a foodstuff. In a further alternative, the composition may be applied as a seasoning, glaze, colorant mixture, and the like.
For some applications, it is important that the composition is made available on or to the surface of a product to be affected/treated. This allows the composition to impart one or more of the following favourable characteristics: nutrition and/or health benefits.
The compositions of the present invention may be applied to intersperse, coat and/or impregnate a product with a controlled amount of a viable microorganism.

### Food

The composition of the present invention may be used as - or in the preparation of - a food. Here, the term "food" is used in a broad sense - and covers food for humans as well as food for animals (i.e. a feed). In a preferred aspect, the food is for human consumption.
The food may be in the form of a solution or as a solid - depending on the use and/or the mode of application and/or the mode of administration.
When used as - or in the preparation of - a food - such as functional food - the composition of the present invention may be used in conjunction with one or more of: a nutritionally acceptable carrier, a nutritionally acceptable diluent, a nutritionally acceptable excipient, a nutritionally acceptable adjuvant, a nutritionally active ingredient.
Preferably, the composition is used to ferment milk or sucrose fortified milk or lactic media with sucrose and/or maltose where the resulting media containing all components of the composition - i.e. said microorganism according to the present invention - can be added as an ingredient to yoghurt milk in suitable concentrations - such as for example in concentrations in the final product which offer a daily dose of 10⁶-10¹⁰ cfu. The microorganism according to the present invention may be used before or after fermentation of the yoghurt. For some aspects the microorganisms according to the present invention or composition according to the present invention are used as - or in the preparation of - animal feeds, such as livestock feeds, in particular poultry (such as chicken) feed, or pet food.

### Food ingredient

The composition of the present invention may be used as a food ingredient and/or feed ingredient.
As used herein the term "food ingredient" or "feed ingredient" includes a formulation which is or can be added to functional foods or foodstuffs as a nutritional supplement.
The food ingredient may be in the form of a solution or as a solid - depending on the use and/or the mode of application and/or the mode of administration.

### Food supplements

The composition of the present invention may be - or may be added to - food supplements.

### Functional foods

The composition of the present invention may be - or may be added to - functional foods.
As used herein, the term "functional food" means food which is capable of providing not only a nutritional effect, but is also capable of delivering a further beneficial effect to consumer.
Accordingly, functional foods are ordinary foods that have components or ingredients (such as those described herein) incorporated into them that impart to the food a specific functional - e.g. medical or physiological benefit - other than a purely nutritional effect.
Although there is no legal definition of a functional food, most of the parties with an interest in this area agree that they are foods marketed as having specific health effects beyond basic nutritional effects.
Some functional foods are nutraceuticals. Here, the term "nutraceutical" means a food which is capable of providing not only a nutritional effect and/or a taste satisfaction, but is also capable of delivering a therapeutic (or other beneficial) effect to the consumer. Nutraceuticals cross the traditional dividing lines between foods and medicine.

### Probiotic

For some applications, it is believed that the viable *Bifidobacterium lactis BL04* in the composition of the present invention can exert a probiotic culture effect. It is also within the scope of the present invention to add to the composition of the present invention further prebiotics.
Here, a prebiotic is:
*"a non-digestible food ingredient that beneficially affects the host by selectively stimulating the growth and*/*or the activity* of *one or a limited number of beneficial bacteria".*
The term "probiotic culture" as used herein defines live microorganisms (including bacteria or yeasts for example) which, when for example ingested or locally applied in sufficient numbers, beneficially affects the host organism, i.e. by conferring one or more demonstrable health benefits on the host organism. Probiotics may improve the microbial balance in one or more mucosal surfaces. For example, the mucosal surface may be the intestine, the urinary tract, the respiratory tract or the skin. The term "probiotic" as used herein also encompasses live microorganisms that can stimulate the beneficial branches of the immune system and at the same time decrease the inflammatory reactions in a mucosal surface, for example the gut.
Whilst there are no lower or upper limits for probiotic intake, it has been suggested that at least 10⁶-10¹², preferably at least 10⁶-10¹⁰, preferably 10⁸-10⁹, cfu as a daily dose will be effective to achieve the beneficial health effects in a host organism, such as a human.
In addition to the probiotic effect the microorganism according to the present invention may have, it is also within the scope of the present invention to provide prebiotics as other compounds which can be included in a combination along with the composition. The prebiotic component of the combination comprising the composition of the present invention are characterised with slow fermentation in the large bowel. Such prebiotics can exert a positive effect on the gut flora, specifically in the left side of the colon, an area of the gut which is especially prone to disorders in particular bowel cancer and ulcerative colitis.
Prebiotics are typically non-digestible carbohydrate (oligo- or polysaccharides) or a sugar alcohol which is not degraded or absorbed in the upper digestive tract. Known prebiotics used in commercial products and useful in accordance with the present invention include inulin (fructo-oligosaccharide, or FOS) and transgalacto-oligosaccharides (GOS or TOS). Other suitable, prebiotics include palatinoseoligosaccharide, soybean oligosaccharide, gentiooligosaccharide, xylooligomers, non-degradable starch, lactosaccharose, lactulose, lactitol, maltitol, polydextrose (i.e. Litesse®) or the like.
In one embodiment the present invention relates to the combination of a *Bifidobacterium lactis BL04* and/or fermentation product therefo and/or cell lysate thereof according to the present invention with a prebiotic.
The prebiotic may be administered simultaneously with (e.g. in admixture together with or delivered simultaneously by the same or different routes) or sequentially to (e.g. by the same or different routes) the microorganism according to the present invention and/or fermentation product therefo and/or cell lysate thereof.

### Synbiotics

The present invention also contemplates using prebiotics as ingredients in a combination along with the composition of the present invention which when combined, become synbiotics. The purpose of this is to combine the effects of the beneficial bacteria and the stimulation of the body-own beneficial bacteria. There is a high potential in the development and the consumption of such mixtures, since some of these may well show powerful synergistic nutritional and/or health effects.
Thus the composition of the present invention may be specifically designed to contain different components which can provide a synbiotic effect to the consumer.

### Pharmaceutical

The composition of the present invention may be used as - or in the preparation of - a pharmaceutical. Here, the term "pharmaceutical" is used in a broad sense - and covers pharmaceuticals for humans as well as pharmaceuticals for animals (i.e. veterinary applications). In a preferred aspect, the pharmaceutical is for human use and/or for animal husbandry.

The pharmaceutical can be for therapeutic purposes - which may be curative or palliative or preventative in nature. The pharmaceutical may even be for diagnostic purposes.
When used as - or in the preparation of - a pharmaceutical, the composition of the present invention may be used in conjunction with one or more of: a pharmaceutically acceptable carrier, a pharmaceutically acceptable diluent, a pharmaceutically acceptable excipient, a pharmaceutically acceptable adjuvant, a pharmaceutically active ingredient.
The pharmaceutical may be in the form of a solution or as a solid - depending on the use and/or the mode of application and/or the mode of administration.

### Pharmaceutical ingredient

The microorganisms of the present invention may be used as pharmaceutical ingredients. Here, the composition may be the sole active component or it may be at least one of a number (i.e. 2 or more) of active components.
The pharmaceutical ingredient may be in the form of a solution or as a solid - depending on the use and/or the mode of application and/or the mode of administration.

### Forms

The microorganism of the present invention and/or fermentation product thereof and/or cell lysate thereof may be used in any suitable form - whether when alone or when present in a combination with other components or ingredients. Likewise, combinations comprising the composition of the present invention and other components and/or ingredients (i.e. ingredients - such as food ingredients, functional food ingredients or pharmaceutical ingredients) may be used in any suitable form.
The microorganism of the present invention or composition of the present invention may be used in the form of solid or liquid preparations or alternatives thereof. Examples of solid preparations include, but are not limited to tablets, capsules, dusts, granules and powders which may be wettable, spray-dried or freeze-dried. Examples of liquid preparations include, but are not limited to, aqueous, organic or aqueous-organic solutions, suspensions and emulsions. Suitable examples of forms include one or more of: tablets, pills, capsules, ovules, solutions or suspensions, which may contain flavouring or colouring agents, for immediate-, delayed-, modified-, sustained-, pulsed- or controlled-release applications.
By way of example, if the composition of the present invention is used in a tablet form - such for use as a functional ingredient - the tablets may also contain one or more of: excipients such as microcrystalline cellulose, lactose, sodium citrate, calcium carbonate, dibasic calcium phosphate and glycine; disintegrants such as starch (preferably corn, potato or tapioca starch), sodium starch glycollate, croscarmellose sodium and certain complex silicates; granulation binders such as polyvinylpyrrolidone, hydroxypropylmethylcellulose (HPMC), hydroxypropylcellulose (HPC), sucrose, gelatin and acacia; lubricating agents such as magnesium stearate, stearic acid, glyceryl behenate and talc may be included.
Examples of nutritionally acceptable carriers for use in preparing the forms include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.
Preferred excipients for the forms include lactose, starch, a cellulose, milk sugar or high molecular weight polyethylene glycols.
For aqueous suspensions and/or elixirs, the composition of the present invention may be combined with various sweetening or flavouring agents, colouring matter or dyes, with emulsifying and/or suspending agents and with diluents such as water, propylene glycol and glycerin, and combinations thereof.
The forms may also include gelatin capsules; fibre capsules, fibre tablets etc.; or even fibre beverages.
Further examples of form is in the form of a cream for example. For some aspects the microorganism and/or a metabolite thereof may be included in pharmaceutical and/or cosmetic creams such as sun creams and/or after-sun creams for example.

In one aspect, the composition according to the present invention may be administered in an aerosol, for example by way of a nasal spray, for instance for administration to the respiratory tract.

**Examples** The present invention will be further described with reference to the following Examples and figures in which:-
Figure 1 shows a schematic representation of the experiments undertaken to show the present invention.
Figure 2 shows a Consort Flow Chart that details the recruitment, processing and analysis of subjects.

### Methodology

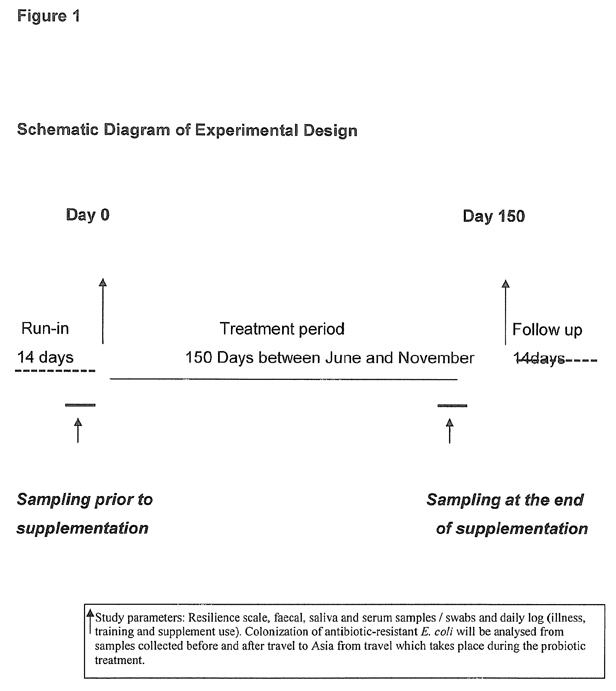

Experimental approach: The study involved a double-blind placebo-controlled trial of healthy physically active individuals from the community to establish whether 150 days supplementing with a probiotics reduces URTI during the winter period between June and October 2010 (Figure 1). There were two experimental groups, a placebo group and a probiotic groups, comprising 309 healthy physically active adults 157 males, 152 females in equivalent numbers. Participants completed a 14 day run-in where all use of probiotic and probiotic supplements / enriched foods and immunomodulatory medications or supplements was stopped. Following baseline sampling, subjects undertook a 150 day supplementation period. All participants were asked to maintain a daily illness diary to record patterns of illness (duration and severity). A cohort of participants was chosen from each group for secondary analysis of immune and microbiology function. Each cohort of participants provided blood and faecal samples, and a throat swab to examine the effect of supplementation on enteric and URT microbiota, indices of innate immune function (NK cell function and phagocytosis). Saliva samples were collected but analysis will be dependent on whether beneficial clinical outcomes are identified. A secondary analysis involved collection of a faecal swab for subjects who travelled to Asia for determination of whether probiotic supplementation reduces the colonization of antibioticresistant *Escherichia coli* during travelling.

Ethics committee clearance was granted by the Ethics Committees of the Australian Institute of Sport (19 February 2010) and Griffith University (11 March 2010).

### Subjects and recruitment

There were 268 healthy, physically active members of the community recruited to the study. Of these, 226 individuals were included in the statistical analysis of the physical activity and illness measures. Subject characteristics of those included in the statistical analysis are detailed in Table 1. There were no substantial differences between the groups.

### Inclusion criteria:

Inclusion to the study was determined according to physical activity levels with participants required to be undertaking a minimum of three exercise sessions weekly.

### Exclusion criteria:

All participants were required to declare their use of dietary and/or ergogenic aids that may influence underlying immune function. All participants on immuno-modulatory medications were excluded, including those on steroid based anti-asthma treatments. Subjects who were on antibiotic treatments in the previous month were also excluded. Subjects with any symptoms of gastrointestinal disease, such as Crohn's disease, coelic disease and related conditions were excluded.

### Primary outcome measures:

The primary clinical outcome measure was URT illness in the participants over the study period. Subjects were required to record any symptoms of URTI and chest illness on a daily illness log over the study period. Briefly, URTI symptoms included throat soreness, sneezing, a blocked or runny nose and cough. Lower respiratory illness symptoms included coughing with chest congestion and/or wheezing. A classification of an episode of illness was made when two or more symptoms were recorded on consecutive days. The functional impact or severity of symptoms for physically active individuals were self-rated as mild, moderate or severe based on the impact of the symptoms on daily activity for that day: mild - no change, moderate -a reduction in normal activity, and severe - total cessation of activity.

### Secondary outcome measures

Perceived stress and resilience: Participant's perceived stress and resilience were measured by questionnaire pre- and post- supplementation. Psychological and social factors represent a source of stress that may affect immunity and health. All subjects undertook the Connor Davidson Resilience questionnaire prior to and at the end of supplementation.

A cohort of participants (53 in the *B.lactis BL-04* group, 51 in the placebo group) from each of the treatment groups provided samples for the following secondary outcome measures.

### Faecal microbiology

- Total bacterial count (eubacteria):
- Quantification of the bacterial groups in fecal samples:
   These groups may include, but are not limited to, *Bacteroidetes, Enterobacteriaceae, Lactobacillus* spp., *Bifidobacterium* spp. *Clostridium* cluster XIV and other clostridial clusters, *Clostridium difficile, Collinsella, Escherichia coli, Enterococcus* spp., *Faecalibacterium prausnitzii, Roseburia* spp., *Veillonella* spp., and sulphate reducing bacteria. Bacterial quantification will be carried out with qPCR and/or with other relevant culture-independent methods. Analysis of the throat swab bacteria by qPCR will focus on particularly relevant bacterial groups, which may include, but are not limited to, *Staphylococcus aureus, Pneumococcus* spp. and *Streptococcus* spp.
- Antibiotic-resistant *Escherichia coli:*
- *L. acidophilus NCFM* and B. *lactis Bi-07* and B. *lactis BL-04:*

### Serum

- Natural killer cell activity
- Phagocytosis

### Data Analysis

A practical approach to making an inference (conclusion) about the clinical and physiological effects of the probiotic treatments was used. This approach has been detailed in several articles (6). This approach is also consistent with the International Committee of Medical Journal Editors guidelines for assessing clinical trials. The merits of this approach to address some of the shortcomings of an approach based on hypothesis testing and statistical significance is well recognised. The approach is based on where the range in uncertainty in the true value of an effect falls in relation to thresholds for values that are clinically important. The uncertainty in the true value is the confidence interval. Because there are a large number of effects in this study, a conservative level of 99% was chosen for the confidence interval; in other words, there is a 99% chance that the true value of each effect falls within the confidence interval that is calculated for it from the data. When the confidence interval includes values that are substantial in some positive and negative sense, such as beneficial and harmful, the effect could be both beneficial and harmful, here it has been inferred that the effect is *inconclusive* or *unclear.* Otherwise it is inferred that the effect is clear, and the magnitude assigned to the effect is the observed magnitude, such as a beneficial, trivial or harmful difference.

The thresholds chosen as clinically important differ for the different kinds of outcome variable in the study. For variables such as the intensity of a symptom on a 3- point scale, the effect of the probiotic treatment was analyzed as a simple difference of the means: probiotic mean minus placebo mean. For this kind of effect the default thresholds are a positive and negative difference in the means equal to 0.20 of the pooled between-subject standard deviation in the two groups. This approach to smallest important effects is known as standardization, and provides thresholds for moderate, large and very large effects (0.60, 1.20 and 2.0 standard deviations). Other variables for which we chose magnitude thresholds in this manner were the number of medications taken per 100 days, the intensity of physical activity, the number of exercise days per week, total exercise hours per week, total activity load per week (sum of the product of exercise intensity and number of exercise days per week), and the variables in saliva samples that were measured for their potential role as mechanisms of any effect of the treatment. The measures of training hours and training load were log-transformed before analysis to permit the effect of the treatment to be properly analyzed as a percent, but magnitude of the effect was determined for the log-transformed variable. The saliva measures were also log transformed before analysis, but variability and effects for these variables were generally much larger than for the training variables and were therefore expressed as factors.

Magnitude thresholds for variables representing or involving the presence or count of a symptom had to be determined in a different manner, because use of a standard deviation for such variables is not appropriate. The variables in question were number of episodes of a given symptom per 100 days, total number of days of the symptom per 100 days, and total load of the symptom per 100 days (sum of the product of symptom intensity and number of days of the symptom per 100 days). The effect of the probiotic treatment on all these variables was analyzed as a ratio: the mean of the probiotic group divided by the mean of the placebo group. We regarded a ratio of 1.10 (that is, a 10% larger mean value of the variable in the probiotic group) as the threshold for a substantial increase. For statistical reasons, the threshold for a substantial decrease in the probiotic group was therefore a ratio of 1/1.2, or 0.83. These ratios are similar to the risk ratios for illness and injury in studies of public health, epidemiologists consider a risk ratio of 1.1-1.3 to represent substantial increase in risk. Unfortunately there is as yet no consensus about thresholds representing moderate, large and very large increases and decreases in risk.

The data from the "shoulder" periods defined as 2 weeks following the start and end of supplementation were analysed using a linear weighting factor to assign an appropriate proportion of the training and symptom scores to the baseline and full treatment periods. Thus, on the first day following the start of treatment, 13/14th of a subject's values were assigned to the baseline period and 1/14th was assigned to the treatment period. On the second day of the shoulder, the fractions were 12/14th and 2/14, and so on.

Data from the baseline was also taken into account. When baseline values are recorded, it is usual to adjust for differences between subjects at baseline by subtracting baseline scores from treatment scores. This strategy usually results in greater precision of the effect of a treatment than that provided by an analysis of the treatment scores alone, and it thereby permits the use of smaller sample sizes. However, it is not generally appreciated that adjusting for a baseline score in this manner results in better precision only when the variable being analyzed is reasonably reliable (that is, subjects' scores tend to be consistently different from each others' in pre and post treatment trials). In this study the symptom and training variables as treatment-only scores were analysed. It was clear that the effect of the treatment on symptoms was more precise for the treatment-only analysis, whereas the effect on training was more precise for the treatment-baseline changes: evidently the subjects' training was more reliable and well defined by the baseline monitoring than their illness symptoms. Therefore, the treatment-only analyses is presented for the symptoms and the treatment-baseline analyses for training. In making the decision about using treatment-only vs treatment-baseline analyses, examining the magnitude of the effect was deliberately avoided, focus was only on comparing the precision of the estimate of the effect of the treatment. The saliva variables have also been presented as treatment-baseline analyses.

Confidence limits for the symptom scores were estimated by an empirical method known as bootstrapping, because the usual analytical approaches involve assumptions that are difficult to justify for measures involving duration of the symptom. Bootstrapping was also used with the training measures. These analyses were performed using programs written in the Statistical Analysis System.

### Treatments

Supplement Z contained a combined *Lactobacillus acidophilus NCFM* and *Bifidobacterium lactis Bi-07 -* the dosage was 5x10⁹ CFU/day for each bacterium, therefore a total dosage of 10⁹ CFU/day.

Supplement X contained *Bifidobacterium lactis BL-04 -* the dosage was 2x10⁹ CFU/day.

The placebo supplement Y contained sucrose.

Supplements Z and X were freeze dried bacteria mixed into a cold drink (no alcohol was to be consumed and the drink was not hot).

### Results

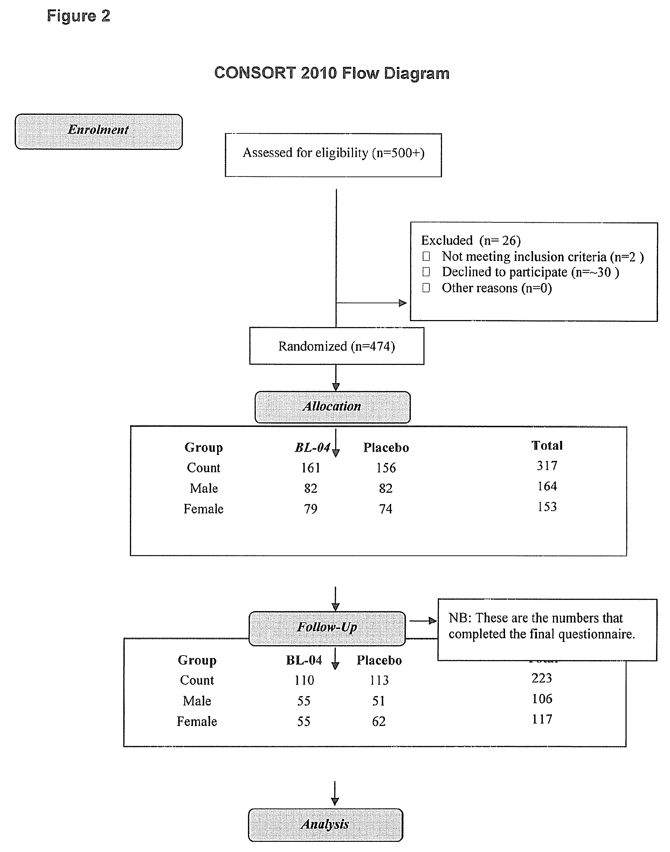

A Consort Flow Chart is presented in Figure 2 that details the recruitment, processing and analysis of subjects.

### Subjects details

Physical and Physiological Characteristics: Details of subject characteristics included for analysis are detailed in Table 1 for each of the groups. There were no substantial differences between the groups in gender, age or body mass index.

**Table 1**

| Characteristics of the individuals included for analysis in each of the groups. Mean ± SD. | | | | | | | |
|---|---|---|---|---|---|---|---|
| Variable | Group | Sex | Number | Mean | SD | Min | Max |
| Age | BL-04 | female | 65 | 39.2 | 11.1 | 19.3 | 61.2 |
| Age | Placebo | female | 66 | 37.2 | 12 | 19.5 | 64.4 |
| | | | | | | | |
| Age | BL-04 | male | 72 | 36 | 11.4 | 18,9 | 65.5 |
| Age | Placebo | male | 65 | 37,7 | 10.1 | 19 | 55.6 |
| | | | | | | | |
| BMI | BL-04 | female | 62 | 24.1 | 3.1 | 18.8 | 32.4 |
| BMI | Placebo | female | 63 | 23.5 | 3.4 | 17.4 | 32.6 |
| | | | | | | | |
| BMI | BL-04 | male | 68 | 24.8 | 2.9 | 14.2 | 31.6 |
| BMI | Placebo | male | 61 | 25.3 | 2.6 | 20.1 | 32.2 |
| | | | | | | | |
| ExerciseHoursPerWk | BL-04 | female | 29 | 9.6 | 5.6 | 2 | 25 |
| ExerciseHoursPerWk | Placebo | female | 34 | 7 | 3.4 | 3 | 16 |
| | | | | | | | |
| ExerciseHoursPerWk | BL-04 | male | 36 | 7.7 | 3.2 | 2.5 | 15.5 |
| ExerciseHoursPerWk | Placebo | male | 31 | 7.9 | 3 | 3.5 | 14 |

### Compliance

Compliance details and the mean number of supplement days completed by participants in each group are detailed in Table 2.

**Table 2**

| The degree of compliance and the mean number of supplement days completed by participants in each of the three treatment groups over the course of the study. | | | | | | |
|---|---|---|---|---|---|---|
| Variable | Group | Sex | Mean | SD | Min | Max |
| % sachets left | BL-04 | female | 13.5 | 17.9 | 0 | 100 |
| % sachets left | Placebo | female | 11.6 | 12.7 | 0 | 55 |
| | | | | | | |
| % sachets left | BL-04 | male | 7.3 | 12.1 | 0 | 75 |
| % sachets left | Placebo | male | 15.1 | 16.1 | 0 | 80 |
| | | | | | | |
| Supplement days (%) | BL-04 | female | 95.7 | 5.8 | 70.6 | 100 |
| Supplement days (%) | Placebo | female | 94.4 | 7.5 | 71.8 | 100 |
| | | | | | | |
| Supplement days (%) | BL-04 | male | 95.2 | 8.4 | 60.5 | 100 |
| Supplement days (%) | Placebo | male | 95 | 6.5 | 73.8 | 100 |

### Adverse effects

Four subjects experience diarrheoa and cramps at the onset of supplementation. Three of these subjects withdrew and the symptoms settled in the third. One subject withdrew due to headaches that started with supplementation, including after a break from taking the supplement assigned. One subject experienced urticaria after starting supplementation and withdrew. One subject experienced bowel pain after travelling to Asia. The date of travel coincided with taking the supplement and the subject withdrew.

### Dietary information

Dietary information regarding fibre intake in each of the treatment groups is in Table 3. There were no substantial differences between the groups in fibre serves.

**Table 3**

| Number of standard serves of fibre per day by treatment group and gender at the midpoint (Mid), end of the study (End) and mean of both time points (Mean). | | | | | | | | |
|---|---|---|---|---|---|---|---|---|
| Variable | Treatment | Sex | N | Nmiss | Mean | SD | Min | Max |
| FiberServesPerDayMid | BL-04 | female | 48 | 17 | 4.0 | 6.1 | 0.3 | 31.8 |
| FiberServesPerDayMid | BL-04 | male | 55 | 17 | 2.9 | 1.6 | 0.3 | 7.3 |
| FiberServesPerDayMid | Pla | female | 56 | 10 | 4.8 | 8.3 | 0.6 | 46.7 |
| FiberServesPerDayMid | Pla | female | 48 | 17 | 4.9 | 8.1 | 0.4 | 56.5 |
| | | | | | | | | |
| FiberServesPerDayEnd | BL-04 | female | 52 | 13 | 3.3 | 4.5 | 0.2 | 32.4 |
| FiberServesPerDayEnd | BL-04 | male | 55 | 17 | 4.0 | 5.5 | 0.3 | 40.8 |
| FiberServesPerDayEnd | Pla | female | 56 | 10 | 4.4 | 6.4 | 0.3 | 40.8 |
| FiberServesPerDayEnd | Pla | female | 53 | 12 | 3.7 | 3.5 | 0.0 | 23.4 |
| | | | | | | | | |
| FiberServesPerDayMean | BL-04 | female | 55 | 10 | 3.6 | 4.6 | 0.3 | 31.4 |
| FiberServesPerDayMean | BL-04 | male | 60 | 12 | 3.4 | 3.0 | 0.3 | 22.4 |
| FiberServesPerDayMean | Pla | female | 62 | 4 | 5.0 | 7.4 | 0.8 | 40.8 |
| FiberServesPerDayMean | Pla | male | 57 | 8 | 4.3 | 4.6 | 0.6 | 30.0 |
| Pla - placebo; | | | | | | | | |

### Physical activity information

Participant training details by treatment group during the study are presented in Table 4. There were no substantial differences between the groups in physical activity patterns.

**Table 4**

| Subject training during the study. Intensity scored on a 1-10 scale; Training load is the sum of the product of training intensity and training duration in arbitrary units. | | | | | | | |
|---|---|---|---|---|---|---|---|
| Variable | Group | Sex | Number | Mean | SD | Min | Max |
| Training intensity | BL-04 | female | 65 | 5.7 | 1.4 | 1.8 | 8.6 |
| Training intensity | Placebo | female | 66 | 5.8 | 1.4 | 1.3 | 8.9 |
| | | | | | | | |
| Training intensity | BL-04 | male | 72 | 6.1 | 1.3 | 2.9 | 9 |
| Training intensity | Placebo | male | 65 | 6.1 | 1.1 | 2.6 | 8.5 |
| | | | | | | | |
| TrainingDaysPerWeek | BL-04 | female | 65 | 4.5 | 1.3 | 2.1 | 6.9 |
| TrainingDaysPerWeek | Placebo | female | 66 | 4.1 | 1.2 | 1.4 | 6.9 |
| | | | | | | | |
| TrainingDaysPerWeek | BL-04 | male | 72 | 3.9 | 1.3 | 1.6 | 6.3 |
| TrainingDaysPerWeek | Placebo | male | 65 | 4 | 1.4 | 0.6 | 6.9 |
| | | | | | | | |
| TrainingHoursPerWeek | BL-04 | female | 65 | 5.9 | 2.6 | 2 | 14.6 |
| TrainingHoursPerWeek | Placebo | female | 66 | 5.4 | 2.9 | 1.5 | 18.2 |
| | | | | | | | |
| TrainingHoursPerWeek | BL-04 | male | 72 | 5.9 | 3.7 | 1.5 | 24.2 |
| TrainingHoursPerWeek | Placebo | male | 65 | 5.7 | 4 | 1 | 19.5 |
| | | | | | | | |
| TrainingLoadPerWeek | BL-04 | female | 65 | 34 | 16 | 8.2 | 79.9 |
| TrainingLoadPerWeek | Placebo | female | 66 | 31.7 | 17.8 | 4.2 | 98.8 |
| | | | | | | | |
| TrainingLoadPerWeek | BL-04 | male | 72 | 35.2 | 19.8 | 4.9 | 119.2 |
| TrainingLoadPerWeek | Placebo | male | 65 | 33.7 | 21.9 | 6.3 | 119.3 |

### Episodes of illness

### Upper Respiratory Tract Illness

The effect of probiotic supplementation on the number of episodes of respiratory tract illness of varying duration is at in Table 5a. B. *lactis BL04* reduced the number of respiratory tract illnesses of longer duration. Furthermore, this effect was more pronounced as episodes of illness became longer.

| **Table 5a: The effect of probiotic treatment on symptoms of upper respiratory tract symptoms.** | | | | | |
|---|---|---|---|---|---|
| **Length of illness** | **Probiotic group (Mean ± SD)** | **Placebo group (Mean ± SD)** | **Effect of probiotic treatment relative to placebo (Mean; 99% CI)** | **Clinical inference** | ***P-*value** |
| 1 day | 1.93 | 1.93 | 1.00 (0.74 to 1.72) | Unclear | 0.9 |
| 3 day | 0.6 | 0.75 | 0.80 (0.52 to 1.21) | Possible ↓ | 0.16 |
| 5 day | 0.22 | 0.33 | 0.65 (0.33 to 1.29) | Likely ↓ | 0.11 |
| 7 day | 0.09 | 0.16 | 0.54 (0.21 to 1.30) | Likely ↓ | 0.09 |

### Chest infection (Lower respiratory tract infection)

The effect of *B. lactis* on chest infection is in Table 5b. Similar to the effects of *B. lactis BL04* on URTI, supplementation reduced the number of episodes of chest infection that lasted for 5 days compared to episodes that lasted of shorter duration.

| **Table 5b: The effect of probiotic treatment on symptoms of chest symptoms.** | | | | | |
|---|---|---|---|---|---|
| **Length of illness** | **Probiotic group (Mean ± SD)** | **Placebo group (Mean ± SD)** | **Effect of probiotic treatment relative to placebo (Mean; 99% CI)** | **Clinical inference** | **P-value** |
| 1 day | 1.41 | 1.49 | 0.94 (0.67 to 1.33) | Unclear | 0.7 |
| 3 day | 0.39 | 0.48 | 0.81 (0.48 to 1.38) | Unclear | 0.3 |
| 5 day | 0.11 | 0.19 | 0.55 (0.24 to 1.27) | Likely ↓ | 0.06 |

### Patterns of illness

The difference in the frequency, duration, severity and combined load of upper respiratory illness between probiotic and placebo groups during the treatment period is shown in Table 6. There was a substantially lower illness load and duration in those taking BL-04 compared to placebo. On a gender basis these reductions were also more pronounced in females than in men.

**Table 6**

| | The effect of probiotic treatment on the number, duration, sevei and combined load of respiratory tract infection. | | | |
|---|---|---|---|---|
| | Observed values (mean ± SD) | | Effect of BL-04 vs Placebo | |
| | BL-04 | Placebo | Mean; CI | Inference |
| **All** | | | | |
| # of episodes (/100 days) | 1.93 ± 1.90 | 1.93 ± 1.90 | Ratio 1.0; 0.74 to 1.34 | Unclear |
| Duration (/100 days) | 2.9 ×/÷ 3.8 | 3.9 ×/÷ 3.4 | Difference (%) 25 -52 to 17 | Possible↓ |
| Severity (1-3 scale) | 1.47 ± 0.42 | 1.48 ± 0.36 | Difference -0.01 -0.15 to 0.12 | Unclear |
| Illness Load | 4.1 ×/÷ **4.1** | 5.7 ×/÷ 5.7 | Difference (%) -27; -55 to 18 | Possible ↓ |
| **Females** | | | | |
| # of episodes (/100 days) | 2.01 ± 1.96 | 2.28 ± 1.96 | Ratio 0.88; 0.59 to 1.32 | Unclear |
| Duration (/100 days) | 2.7 ×/÷ 4.6 | 3.0 ×/÷ 3.1 | Difference (%) -44; -71 to 10 | Likely ↓ |
| Severity (1-3 scale) | 1.43 ± 0.43 | 1.44 ± 0.45 | Difference -0.01; -0.18 to 0.17 | Unclear |
| Illness Load | 3.5 ×/÷ 5.2 | 6.3 ×/÷ 3.5 | Difference (%) -45; -73 to 14 | Likely ↓ |
| **Males** | | | | |
| # of episodes (/100 days) | 1.84 ± 1.84 | 1.63 ± 1.69 | Ratio 1.13; 0.74 to 1.72 | Unclear |
| Duration (/100 days) | 3.4 ×/÷ 3.0 | 3.4 ×/÷ 3.4 | Difference (%) -1; -46 to 83 | Unclear |
| Severity (1-3 scale) | 1.51 ± 0.41 | 1.53 ± 0.61 | Difference -0.02; -0.23 to 0.19 | Unclear |
| Illness Load | 4.9 ×/÷ 3.3 | 5.1 ×/÷ 3.4 | Difference (%) -4; -50 to 84 | Unclear |

The difference in the frequency, duration, severity and combined load of chest illness (lower respiratory illness) between probiotic and placebo groups over the treatment period is shown in Table 7. There is a reduction in severity of chest infection symptoms in BL-04 compared to placebo.

**Table 7**

| The effect of probiotic treatment on the number, duration, severity and combined load of Chest infection. | | | | |
|---|---|---|---|---|
| | Observed values (mean ± SD) | | Effect of BL-04 vs Placebo | |
| | BL-04 | Placebo | Mean; CI | Inference |
| **All** | | | | |
| # of episodes (/100 days) | 1.41 ± 1.62 | 1.49 ± 1.70 | Ratio 0.94; 0.67 to 1.33 | Unclear |
| Duration (/100 days) | 2.6 ×/÷ 2.9 | 2.8 ×/÷ 3.1 | Difference (%) -9 -40 to 38 | Unclear |
| Severity (1-3 scale) | 1.42 ± 0.42 | 1.51 ± 0.53 | Difference -0.09 -0.27 to 0.09 | Possible ↓ |
| Illness Load | 3.5 ×/÷ 3.2 | 4.0 ×/÷ 3.3 | Difference (%) -12; 43 to 36 | Unclear |
| **Females** | | | | |
| # of episodes (/100 days) | 1.44 ± 1.66 | 1.80 ± 1.96 | Ratio 0.80; 0.50 to 1.29 | Unclear |
| Duration (/100 days) | 2.7 ×/÷ 2.7 | 3.0 ×/÷ 3.1 | Difference (%) -11; -49 to 55 | Unclear |
| Severity (1-3 scale) | 1.44 ± 0.43 | 1.46 ± 0.45 | Difference -0.01; -0.24 to 0.21 | Unclear |
| Illness Load | 3.8 ×/÷ 3.0 | 4.2 ×/÷ 3.3 | Difference (%) -9.8; -50 to 63 | Unclear |
| **Males** | | | | |
| # of episodes (/100 days) | 1.37 ± 1.59 | 1.24 ± 1.48 | Ratio 1.10; 0.68 to 1.81 | Unclear |
| Duration (/100 days) | 2.5 ×/÷ 3.2 | 2.7 ×/÷ 3.2 | Difference (%) -7; -50 to 74 | Unclear |
| Severity (1-3 scale) | 1.40 ± 0.41 | 1.57 ± 0.61 | Difference -0.17; -0.45 to 0.11 | Unclear |
| Illness Load | 3.3 ×/÷ 3.5 | 3.9 ×/÷ 3.3 | Difference (%) -14; -55 to 65 | Unclear |

The difference in the frequency, duration, severity and combined load of medication usage between the groups over the treatment period is shown in Table 8. Briefly, participants on BL-04 had a substantially lower total number of medications and total days of medications compared to those on the placebo. When examined by gender the effect was maintained in the men but less so in women.

**Table 8**

| The effect of probiotic treatment on the number, duration, severity and combined load of Medication episodes. | | | | |
|---|---|---|---|---|
| | Observed values (mean ± SD) | | Effect of BL-04 vs Placebo | |
| | BL-04 | Placebo | Mean; CI | Inference |
| **All** | | | | |
| # of med episodes (/100 days) | 1.04 ± 1.27 | 1.19 ± 1.39 | Ratio 0.88; 0.62 to 1.25 | Unclear |
| Total days of medications (/100drays) | 2.7 /÷ 3.0 | 4.0 /÷ 3.0 | Difference (%) -31; -55 to 4.4 | Likely small ↓ |
| Mean # of medication per episode | 1.30 ± 0.56 | 1.28 ± 0.44 | Difference 0.01; -0.19 to 0.21 | Unclear |
| Total # of medications | 3.4 /÷ 3.4 | 5.0 /÷ 3.3 | Difference (%) -32; -57 to 9 | Likely ↓ |
| **Females** | | | | |
| # of med episodes (/100 days) | 1.32 ± 1.50 | 1.43 ± 1.58 | Ratio 1.08; 0.67 to 1.73 | Unclear |
| Total days of medications (/100days) | 3.5 /÷ 2.6 | 4.2 /÷ 3.5 | Difference (%) -15; -54 to 58 | Unclear |
| Mean # of medication per episode | 1.32 ± 0.40 | 1.31 ± 0.35 | Difference 0.01; 0.21 to -0.20 | Unclear |
| Total # of medications | 4.6 /÷ 3.0 | 5.5 /÷ 4.0 | Difference (%) -16; -58 to 68 | Unclear |
| **Males** | | | | |
| # of med episodes (/100 days) | 0.82 ± 1,08 | 0.99 ±1.23 | Ratio 0.83; 0.49 to 1.40 | Unclear |
| Total days of medications (/100drays) | 2.1 /÷ 3.2 | 3.8 /÷ 2.4 | Difference (%) -44; -68 to -2 | Very likely ↓ |
| Mean # of medication per episode | 1.27 ± 0.69 | 1.25 ± 0.52 | Difference 0.02; -0.32 to 0.36 | Unclear |
| Total # of medications | 2.5 /÷ 3.5 | 4.5 /÷ 2.6 | Difference (%) -44; -70 to 5 | Likely ↓ |

### Conclusions

The results show that supplementing with *B.lactis BL-04* elicited a substantial decrease in the number of upper and lower respiratory illnesses and a decrease in the severity and duration of URTI and lower respiratory tract symptoms.

### Full data analysis

Interim data analysis at 99% confidence interval suggested that B. lactis BL-04 may be effective in preventing respiratory tract infections and reduce the need of medications associated with respiratory infections (see Tables 5a, 5b, 6, 7 and 8). The encouraging interim results warranted full data analysis, as shown below.

The full data analysis (n=399) was based on the determination of both clinical significance as well as statistical significance testing. The statistical significance was determined using the traditional 95% confidence intervals. For the clinical significance testing, the pre-defined threshold for clinical relevancy was set at 20% reduction of symptoms as compared to the placebo group.

As shown in Table 9, the treatment with B. lactis BI-04 reduced the symptoms of upper respiratory tract illness markedly. The reducing effect was stronger for the illnesses with longer duration, i.e. the more severe episodes of illness. The illnesses with duration of 7 days or more were reduced by 46% as compared to placebo. In all categories of illness duration (3d, 5d, and 7 day or more), the reduction was equal or more than the pre-defined cut-off value of clinical significance. Surprisingly, the effects of BL-04 on upper respiratory illness were stronger than for the combination of NCFM and Bi-07. This was particularly surprising because the BL-04 probiotic was administered at lower dose than the probiotic combination.

Both BL-04 and the combination of NCFM and Bi-07 were equally effective in reducing the lower respiratory chest infections (see Table 9). A separate analysis also showed that both the combined NCFM + Bi-07 as well as BL-04 as single strain had a significant reducing effect on the duration of the illness episodes.

**Table 9:**

| | | | |
|---|---|---|---|
| | | BI-04 vs Placebo (%) | NCFM + Bi07 vs placebo |
| Duration | | Mean; 95% CI % reduction | Mean; 95% CI Rate ratios |

| **Upper respiratory tract illness** | | | |
|---|---|---|---|
| 3 day | | **20%** (-10 to 42%) | 16% (-15 to 39%) |
| 5 day | | **35%** (-9 to 61%) | 19% (-27 to 48%) |
| 7 day | | **46%** (-7 to 73%) | **33%** (-57 to 71%) |

| **Chest illness** | | | |
|---|---|---|---|
| 3 day | | 19% (-21 to 44%) | **33%** (-17 to 49%) |
| 5 day | | **45%** (-4 to 71%) | **53%** (6 to 79%) |

| **Cold and flu medication usage** | | | |
|---|---|---|---|
| 3 day | | **28%** (0 to 49%) | **33%** (-7 to 45%) |
| 5 day | | **45%** (13 to 65%) | **35%** (-3 to 76%) |

Table 9: The effect of probiotic supplements on the rate of upper and lower respiratory tract illnesses, and cold and flu medication usage, stratified by illness duration. Effects exceeding the pre-defined cut-off value of clinical significance (20% reduction) are marked in bold. The BI-04 group had higher reduction of the illness rate vs placebo than the combined NCFM + Bi-07 group vs placebo.

### References

1. Albers, R., J. M. Antoine, R. Bourdet-Sicard, P. C. Calder, M. Gleeson, B. Lesourd, S. Samartin, I. R. Sanderson, J. Van Loo, F. W. Vas Dias, and B. Watzl. Markers to measure immunomodulation in human nutrition intervention studies. Br J Nutr. 94:452-481, 2005.
2. Berg, A., H. M. Muller, S. Rathmann, and P. Deibert. The gastrointestinal system--an essential target organ of the athlete's health and physical performance. Exerc Immunol Rev. 5:78-95, 1999.
3. Cox, A. J., D. B. Pyne, P. U. Saunders, R. Callister, and M. Gleeson. Cytokine responses to treadmill running in healthy and illness-prone athletes. Med Sci Sports Exerc. 39:1918-1926, 2007.
4. Cox, A. J., D. B. Pyne, P. U. Saunders, and P. A. Fricker. Oral administration of the probiotic Lactobacillus fermentum VRI-003 and mucosal immunity in endurance athletes. Br J Sports Med, 2008.
5. de Vrese, M. and J. Schrezenmeir. Probiotics and non-intestinal infectious conditions. Br J Nutr. 88 Suppl 1:S59-66, 2002.
6. de Vrese, M. and J. Schrezenmeir. Probiotics, Prebiotics, and Synbiotics. Adv Biochem Eng Biotechnol, 2008.
7. de Vrese M et al., Probiotic bacteria reduced duration and severity but not the incidence of common cold episodes in a double blind, randomized, controlled trial, Vaccine, 2006 Nov 10, vol. 24, no. 44-46, p6670 (Epub 2006 Jun 6)
8. de Vrese, M., P. Winkler, P. Rautenberg, T. Harder, C. Noah, C. Laue, S. Ott, J. Hampe, S. Schreiber, K. Heller, and J. Schrezenmeir. Effect of Lactobacillus gasseri PA 16/8, Bifidobacterium longum SP 07/3, B. bifidum MF 20/5 on common cold episodes: a double blind, randomized, controlled trial. Clin Nutr. 24:481-491, 2005.
9. Dixon R E., Economic costs of respiratory tract infections in the United States, Am J Med, 1985, vol. 78, no. 6B, 45-51
10. Fricker, P. A., D. B. Pyne, P. U. Saunders, A. J. Cox, M. Gleeson, and R. D. Telford. Influence of training loads on patterns of illness in elite distance runners. Clin J Sport Med. 15:246-252, 2005.
11. Genton, L., W. van Gemert, C. Pichard, and P. Soeters. Physiological functions should be considered as true end points of nutritional intervention studies. Proc Nutr Soc. 64:285-296, 2005.
12. Gleeson, M., W. A. McDonald, D. B. Pyne, A. W. Cripps, J. L. Francis, P. A. Fricker, and R. L. Clancy. Salivary IgA levels and infection risk in elite swimmers. Med Sci Sports Exerc. 31:67- 73, 1999.
13. Gleeson, M., D. B. Pyne, and R. Callister. Exercise effects on mucosal immunity and risk of upper respiratory illness. International Sports Medicine Journal. 4:1-14, 2003.
14. Hashem M and Hall C B, Respiratory syncytial virus in healthy adults: the cost of a cold, Journal of Clinical Virology, 2003, vol 27, no. 1, p14-21
15. Kang, W. and K. A. Kudsk. Is there evidence that the gut contributes to mucosal immunity in humans? JPEN J Parenter Enteral Nutr. 31:246-258, 2007.
16. Kekkonen, R. A., T. J. Vasankari, T. Vuorimaa, T. Haahtela, I. Julkunen, and R. Korpela. The effect of probiotics on respiratory infections and gastrointestinal symptoms during training in marathon runners. Int J Sport Nutr Exerc Metab. 17:352-363, 2007.
17. Koh, D., Y. Yong, V. Ng, and S. E. Chia. Stress, mucosal immunity, upper respiratory tract infections, and sickness absence. J Occup Environ Med. 44:987-988, 2002.
18. Leder K et al., A community-based study of respiratory episodes in Melbourne, Australia, Aust. N Z J Public Health, 2003, vol. 27, no. 4, p399-404
19. Müns, G., P. Singer, F. Wolf, and I. Rubinstein. Impaired nasal mucociliary clearance in longdistance runners. Int J Sports Med. 16:209-213, 1995.
20. Peters, H. P., W. R. De Vries, G. P. Vanberge-Henegouwen, and L. M. Akkermans. Potential benefits and hazards of physical activity and exercise on the gastrointestinal tract. Gut. 48:435- 439, 2001.
21. Pregliasco F et al., A new chance of preventing winter diseases by the administration of synbiotic formulations, J Clin Gastroenterol., 2008 Sep., vol. 42, suppl 3, part 2, S224-233
22. Pyne, D. B. and M. Gleeson. Effects of intensive exercise training on immunity in athletes. Int J Sporfs Med. 19 Suppl 3:S183-191; discussion S191-184, 1998.
23. Pyne, D. B., W. G. Hopkins, A. M. Batterham, M. Gleeson, and P. A. Fricker. Characterising the individual performance responses to mild illness in international swimmers. Br J Sports Med. 39:752-756, 2005.
24. Pyne, D. B., W. A. McDonald, M. Gleeson, A. Flanagan, R. L. Clancy, and P. A. Fricker. Mucosal immunity, respiratory illness, and competitive performance in elite swimmers. Med Sci Sports Exerc. 33:348-353, 2001.
25. Saavedra, J. M. and A. Tschernia. Human studies with probiotics and prebiotics: clinical implications. Br J Nutr. 87 Suppl 2:S241-246, 2002.
26. Welfare, 2006 Australian Institute of Health and Welfare 2006, Australia's Health 2006, Australia's health no. 10 Cat. No. AUS 73. Canberra: AIHW. Page 107
27. West, N. P., D. B. Pyne, J. M. Kyd, G. M. C. Renshaw, P. Fricker, and A. W. Cripps. The effect of exercise on innate mucosal immunity. British Journal of Sports Medicine, 2008.
28. West, N. P., D. B. Pyne, G. Renshaw, and A. W. Cripps. Antimicrobial peptides and proteins, exercise and innate mucosal immunity. FEMS Immunol Med Microbiol. 48:293-304, 2006.
29. Winkler P et al., Effect of a dietary supplement containing probiotic bacteria plus vitamins and minerals on common cold infections and cellular immune parameters, Int. J Clin. Pharmacol. Ther., 2005 Jul, vol. 43, no. 7, p318-326

## Claims

1. A composition comprising a bacterium and/or a fermentation product of a bacterium and/or a cell lysate of a bacterium for use in the treatment or prophylaxis of respiratory tract illness in a subject, wherein said bacterium is *Bifidobacterium lactis BL04* alone.

2. The composition for use according to claim 1, wherein the respiratory tract illness is an upper respiratory tract illness.

3. The composition for use according to claim 1, wherein the respiratory tract illness is a lower respiratory tract illness.

4. The composition for use according to claim 1 or claim 2, wherein the respiratory tract illness is selected from one or more of the group consisting of: tonsillitis, otitis media; rhinitis; rhinosinusitis, sinusitis; nasopharyngitis, rhinopharyngitis, the common cold; pharyngitis; epiglottitis; supraglottitis; laryngitis; laryngotracheitis; tracheitis; throat soreness; sneezing; blocked nose; runny nose and a cough

5. The composition for use according to claim 1 or claim 3 wherein the respiratory tract illness is selected from one or more of the group consisting of: bronchitis; acute bronchitis; pneumonia; a lung abscess; coughing with chest congestion and coughing with wheezing.

6. The composition for use according to any one of the preceding claims, wherein granulocyte phagocytic activity in the subject is increased.

7. The composition for use according to any one of the preceding claims, wherein monocyte phagocytic activity in the subject is increased.

8. The composition for use according to any one of claims 1 to 7, wherein the use is prophylactic.

## Patentansprüche

1. Zusammensetzung, die ein Bakterium und/oder ein Fermentationsprodukt eines Bakteriums und/oder ein Zelllysat eines Bakteriums umfasst, zur Verwendung in der Behandlung oder Prophylaxe von Atemwegserkrankungen in einem Patienten, wobei es sich bei dem Bakterium ausschließlich um *Bifidobacterium lactis BL04* handelt.

2. Zusammensetzung zur Verwendung nach Anspruch 1, wobei es sich bei der Atemwegserkrankung um eine Erkrankung der oberen Atemwege handelt.

3. Zusammensetzung zur Verwendung nach Anspruch 1, wobei es sich bei der Atemwegserkrankung um eine Erkrankung der unteren Atemwege handelt.

4. Zusammensetzung zur Verwendung nach Anspruch 1 oder Anspruch 2, wobei die Atemwegserkrankung aus einer oder mehreren der Gruppe bestehend aus Mandelentzündung, Otitis media; Rhinitis; Rhinosinusitis, Sinusitis; Nasopharyngitis, Rhinopharyngitis, Schnupfen; Pharyngitits, Epiglottitis; Supraglottitis; Laryngitis; Laryngotracheitis; Tracheitis; Halsweh; Niesen; verstopfter Nase; laufender Nase und Husten ausgewählt ist.

5. Zusammensetzung zur Verwendung nach Anspruch 1 oder Anspruch 3, wobei die Atemwegserkrankung aus einer oder mehreren der Gruppe bestehend aus Bronchitis; akuter Bronchitis; Lungenentzündung; Lungenabszess; Husten mit Druck auf der Brust sowie Husten mit pfeifendem Atem ausgewählt ist.

6. Zusammensetzung zur Verwendung nach einem der vorhergehenden Ansprüche, wobei die phagozytäre Aktivität der Granulozyten in dem Patienten erhöht ist.

7. Zusammensetzung zur Verwendung nach einem der vorhergehenden Ansprüche, wobei die phagozytäre Aktivität der Monozyten in dem Patienten erhöht ist.

8. Zusammensetzung zur Verwendung nach einem der Ansprüche 1 bis 7, wobei die Verwendung prophylaktisch ist.

## Revendications

1. Composition comprenant une bactérie et/ou un produit de fermentation d'une bactérie et/ou un lysat de cellules d'une bactérie pour utilisation dans le traitement ou la prophylaxie d'une maladie des voies respiratoires chez un sujet, ladite bactérie étant *Bifidobacterium lactis BL04* seule.

2. Composition pour utilisation selon la revendication 1, **caractérisée en ce que** la maladie des voies respiratoires est une maladie des voies respiratoires supérieures.

3. Composition pour utilisation selon la revendication 1, **caractérisée en ce que** la maladie des voies respiratoires est une maladie des voies respiratoires inférieures.

4. Composition pour utilisation selon la revendication 1 ou revendication 2, **caractérisée en ce que** la maladie des voies respiratoires est choisie parmi une ou plusieurs du groupe constitué de : l'amygdalite, l'otite moyenne ; la rhinite ; la rhinosinusite, la sinusite ; la nasopharyngite, la rhinopharyngite, le rhume ; la pharyngite ; l'épiglottite ; la supraglottite ; la laryngite ; la laryngotrachéite ; la trachéite ; le mal de gorge ; les éternuements ; le nez bouché ; le nez qui coule et une toux.

5. Composition pour utilisation selon la revendication 1 ou la revendication 3 **caractérisée en ce que** la maladie des voies respiratoires est choisi parmi une ou plusieurs du groupe constitué de : la bronchite ; la bronchite aiguë ; la pneumonie ; un abcès pulmonaire ; la toux avec congestion thoracique et la toux avec sifflement.

6. Composition pour utilisation selon l'une quelconque des revendications précédentes, **caractérisée en ce que** l'activité phagocytaire des granulocytes chez le sujet est augmentée.

7. Composition pour utilisation selon l'une quelconque des revendications précédentes, **caractérisée en ce que** l'activité phagocytaire des monocytes chez le sujet est augmentée.

8. Composition pour utilisation selon l'une quelconque des revendications 1 à 7, **caractérisée en ce que** l'utilisation est prophylactique.
